# EUROPEAN PATENT APPLICATION

(11) **EP 2 762 079 A1**
(43) Date of publication of application: **06.08.2014**
(21) Application number: 12835656.5
(22) Date of filing: 27.07.2012
(51) Int. Cl.: A61B 6/00

(54) **DIAGNOSTIC X-RAY IMAGING EQUIPMENT AND X-RAY IMAGE DISPLAY METHOD**

(30) Priority: 30.09.2011 JP 2011217668
(71) Applicant: Hitachi Medical Corporation, Chiyoda-ku Tokyo 101-0021 (JP)
(72) Inventor: TSUKIJISHIN, Kenta, Chiyoda-ku, Tokyo 100-8280 (JP); MOTOKI, Kouhei, Chiyoda-ku, Tokyo 101-0021 (JP)
(74) Representative: Strehl Schübel-Hopf & Partner
(86) International application number: PCT/JP2012/069243
(87) International publication number: WO 2013/046901

(57) **Abstract**

There is provided diagnostic X-ray imaging equipment that can reproduce only a desired image range at a desired reproduction speed among a series of consecutive images for an operator. A first band-shaped region 44 of which an overall length corresponds to an overall length of time of consecutive X-ray images in chronological order and a second band-shaped region 44 in which a plurality of positions in a length direction respectively correspond to a plurality of types of reproduction speed determined in advance are displayed on a display device. In a case where an operator designates a position in the first band-shaped region 44, the X-ray image corresponding to the designated position among the consecutive X-ray images is selected to be displayed on the display device. In a case where the operator designates a position in the second band-shaped region 45, the consecutive X-ray images in chronological order among the X-ray images which are selected to be displayed at a speed corresponding to the designated position are read out to be displayed on the display device.

## Description

### Technical Field

The present invention relates to diagnostic X-ray imaging equipment, and particularly relates to equipment capable of displaying a desired consecutive range among a large number of consecutive images at a desired display speed for an operator.

### Background Art

A contrast agent is injected into the blood vessel of a test subject to perform X-ray cineradiography (angiography) in which X-ray projection is continuously carried out. A large number of acquired consecutive images (cinema images) are recorded in an image memory. These images are read out from the image memory after image-capturing by an operation of an operator to be displayed in still images or consecutively read out for a moving image display (cinema image display).

Patent Citation 1 discloses that during a moving image display of cinema images, the operator turns a knob to select either a still image display or the moving image display, select a reproduction speed (updating speed) of the moving image, and control a reproduction direction (whether or not reproduction is in reverse). Signs are marked outside the knob indicating a display speed, a pause and a display speed of the reverse reproduction. The operator turns the knob to adjust to a position of a desired mark, thereby making a selection.

In addition, Patent Citation 2 and the like discloses a known method, for example, in which a peak contrast of each pixel in a plurality of images is detected and held to add the plurality of images and to obtain the images of the entirety of the blood vessels by superposing images of the contrast agent which flows in the blood vessels.

### Related Art Documents

### Citation List

[Patent Citation 1] JP-A-6-165188 (particularly, Fig. 3)
[Patent Citation 2] JP-A-6-165035

### Disclosure of Invention

### Technical Problem

In angiography, it is necessary to consecutively capture a state where a contrast agent flows into a blood vessel for a predetermined imaging region and flows out, thereby starting image-capturing before the contrast agent flows in and ending the image-capturing after the contrast agent flows out. For this reason, since multiple images in the beginning are captured before the contrast agent flows in, there is no trace of the captured contrast agent in a series of images, and since multiple images in the end also are captured after the contrast agent flows out, there is no trace of the captured contrast agent in the series of images.

However, when reproducing the moving image, since these images with no trace of the captured contrast agent also are reproduced in a series of the images, before and after the consecutive images with the contrast agent flowing which is desired to be actually seen by an operator, the images with no trace of the contrast agent also are reproduced. The operator repeatedly reproduces the same consecutive images several times as changing reproduction conditions such as a reproduction speed, presence or absence of a peak hold and the like, thereby understanding a shape of a blood vessel and a state of the flowing contrast agent and performing a diagnosis. Even if the time for one reproduction of a series of the images takes several tens of seconds to several minutes, the reproduction is repeated until the diagnosis is made, thereby wasting time during the reproduction mode. For this reason, equipment that can repeatedly reproduce only the image within a desired time range at a desired reproduction speed in a short period by a simple operation is desired.

An object of the present invention is to provide diagnostic X-ray imaging equipment that can reproduce only the desired image range at the desired reproduction speed among a series of the consecutive images for the operator.

### Technical Solution

In order to achieve the above-described object, according to the present invention, there is provided diagnostic X-ray imaging equipment as follows. This equipment includes a storage device that stores X-ray images continuous in chronological order; and a display control unit that controls reproduction of the X-ray image. The display control unit causes a first band-shaped region of which an overall length corresponds to an overall length of time of consecutive X-ray images and a second band-shaped region in which a plurality of positions in a length direction respectively correspond to a plurality of types of reproduction speed determined in advance to be displayed on a display device. In addition, in a case where an operator designates a position in the first band-shaped region, the display control unit selects an X-ray image corresponding to the designated position among the consecutive X-ray images to display on the display device. In a case where the operator designates a position in the second band-shaped region, the display control unit reads out consecutive X-ray images in chronological order among the X-ray images which are selected to be displayed at a speed corresponding to the designated position to display on the display device.

### Advantageous Effects

According to the present invention, an operator can designate a desired image among a series of consecutive images in a first band-shaped region and designate a desired reproduction speed in a second band-shaped region, and thus, it is possible to easily reproduce the desired image range in the desired reproduction speed.

### Brief Description of Drawings

[Fig. 1] Fig. 1 is a block diagram illustrating an overall configuration of diagnostic X-ray imaging equipment according to an embodiment.
[Fig. 2] Fig. 2 is a functional block diagram illustrating a portion of a function of a control device 413 in Fig. 1.
[Fig. 3] Fig. 3 is a flow chart illustrating an operation of a display control unit 11.
[Fig. 4] Figs. 4(a) and 4(b) are explanatory views illustrating examples of a screen of a display unit of an operation table 407.
[Fig. 5] Figs. 5(a) and 5(b) are explanatory views illustrating a track bar 44 to operate image reproduction on the screen of the display unit of the operation table 407.
[Fig. 6] Figs. 6(a) to 6(c) are explanatory views illustrating a slider 45 to operate reproduction speed on the screen of the display unit of the operation table 407.
[Fig. 7] Fig. 7 is an explanatory view illustrating another aspect of the track bar 44 for operating image reproduction on the screen of the display unit of the operation table 407.
[Fig. 8] Fig. 8 is a flow chart illustrating another operation of the display control unit 11.
[Fig. 9] Figs. 9(a) to 9(c) are explanatory views illustrating another aspect of the track bar 44 for operating image reproduction on the screen of the display unit of the operation table 407.
[Fig. 10] Figs. 10(a) and 10(b) are explanatory views illustrating another aspect of the slider 45 for operating reproduction speed on the screen of the display unit of the operation table 407.
[Fig. 11] Fig. 11 is an explanatory view illustrating another aspect of the slider 45 for operating reproduction speed on the screen of the display unit of the operation table 407.
[Fig. 12] Fig. 12 is an explanatory view illustrating an example of the screen of the display unit of the operation table 407.
[Fig. 13] Fig. 13 is a flow chart illustrating another operation of the display control unit 11.
[Fig. 14] Fig. 14(a) is an example of a reproduction image displayed on the display unit of the operation table 407, and Fig. 14(b) is an example of a peak hold image.
[Fig. 15] Fig. 15 is a flow chart illustrating another operation of the display control unit 11.
[Fig. 16] Fig. 16 is a flow chart illustrating another operation of the display control unit 11.
[Fig. 17] Fig. 17 is a flow chart illustrating another operation of the display control unit 11.
[Fig. 18] Fig. 18 is a flow chart illustrating another operation of the display control unit 11.
[Fig. 19] Fig. 19 is a flow chart illustrating another operation of the display control unit 11.
[Fig. 20] Fig. 20 is a flow chart illustrating another operation of the display control unit 11.

### Best Mode for Carrying Out the Invention

Diagnostic X-ray imaging equipment according to the present invention includes a storage device that stores X-ray images continuous in chronological order; and a display control unit that controls reproduction of the X-ray image. The display control unit causes a first band-shaped region of which an overall length corresponds to an overall length of time of consecutive X-ray images and a second band-shaped region in which a plurality of positions in a length direction respectively correspond to a plurality of types of reproduction speed determined in advance to be displayed on a display device. In addition, in a case where an operator designates a position in the first band-shaped region, the display control unit selects the X-ray image corresponding to the designated position among the consecutive X-ray images to display on the display device. In a case where the operator designates a position in the second band-shaped region, the display control unit reads out the consecutive X-ray images in chronological order among the X-ray images which are selected to be displayed at a speed corresponding to the designated position to display on the display device.

Accordingly, it is possible to reproduce only the desired image range at the desired reproduction speed among a series of the consecutive images for the operator.

For example, the display control unit is configured to cause a reproduction start image registration button and a reproduction end image registration button which register the consecutive images within a predetermined time range among the X-ray images continuous in chronological order to be displayed on the display device. In a case where the operator operates the reproduction start image registration button, the display control unit causes the X-ray image which is displayed on the display device at that time to be registered with a storage device as a reproduction start image of the consecutive images within a predetermined time range. In a case where the operator operates the reproduction end image registration button, the display control unit causes the X-ray image which is displayed on the display device at that time to be registered with the storage device as a reproduction end image of the consecutive images within a predetermined time range.

In addition, for example, the display control unit may cause a reproduction button which instructs reproduction to be displayed on the display device. In a case where the operator operates the reproduction button, the display control unit sequentially reproduces reproduction images from the reproduction start image through the reproduction end image which are registered with the storage device. In a case where the operator designates a position in the second band-shaped region during the reproduction mode, the display control unit changes the reproduction speed to a speed corresponding to the designated position.

In addition, the display control unit may cause a slider button for the operator designating a position in the second band-shaped region to be displayed. In a case where the operator continues an operation designating the position in the second band-shaped region using the slider button, the display control unit causes the slider button to stop at the designated position, and in a case where the operator finishes the operation of the slider button, the display control unit causes the slider button to return to the position determined in advance in the second band-shaped region.

The display control unit also may display predetermined marks at positions in the first band-shaped region respectively corresponding to a time position of the reproduction start image and the reproduction end image.

The display control unit may be configured to cause a second reproduction button with which the operator instructs reproduction to be displayed in the first band-shaped region. In this case, the display control unit moves the second reproduction button to a position in the first band-shaped region corresponding to a time position of the X-ray image which is currently displayed on the display device.

It is also preferable for the diagnostic X-ray imaging equipment to be configured to further include a peak hold image generation unit that generates a peak hold (a function of "peak hold" includes "peak opacification") image by sequentially adding the X-ray images. In this case, the display control unit causes a peak hold button which receives an instruction to generate the peak hold image from the operator to be displayed on the display device. In a case where the operator operates the peak hold button, the display control unit causes reproduction images from the reproduction start image through the reproduction end image to be sequentially transferred to the peak hold image generation unit and causes the peak hold images by that time to be sequentially generated, thereby consecutively display the generated peak hold images.

Hereinafter, diagnostic X-ray imaging equipment according to an embodiment of the present invention will be described in detail.

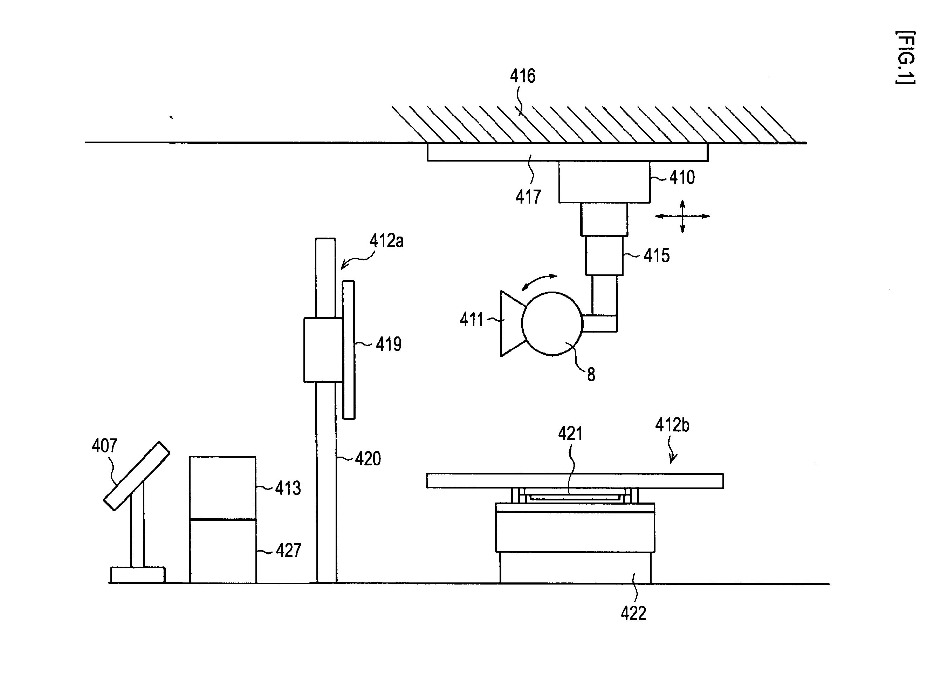

Firstly, an overall configuration of the diagnostic X-ray imaging equipment according to the embodiment will be described with reference to Fig. 1. As illustrated in Fig. 1, the diagnostic X-ray imaging equipment includes an X-ray tube 8, an X-ray tube support device 410, a collimator 411, X-ray image receiving devices 412a and 412b, an X-ray high-voltage device 427, a control device 413 and an operation table 407. The X-ray tube 8 is a device which generates an X-ray being irradiated to a test subject. The X-ray tube support device 410 has a mechanism portion and an expansion portion 415. The mechanism portion changes an irradiation direction of the X-ray from the X-ray tube 8. In addition, the X-ray tube support device 410 is configured to be attached to a rail 417 which is disposed on a ceiling 416 to be movable along the rail 417. The collimator 411 determines an irradiation field of the X-ray. The X-ray high-voltage device 427 supplies set tube voltage and tube current to the X-ray tube 8.

The X-ray image receiving devices 412a and 412b are devices which receive the X-ray penetrating the test subject. There are disposed two devices in the embodiment. The X-ray image receiving device 412a is used to capture the image of the test subject in a standing position. The X-ray image receiving device 412b is used to capture the image of the test subject in a recumbent position. The X-ray image receiving device 412a for the standing position has an image-capturing unit 419 assembled with an image intensifier and a TV camera, and a support structure 420 supporting the image-capturing unit 419. The X-ray image receiving device 412b for the recumbent position has a bed 422 in which an image-capturing unit 421 assembled with the image intensifier and the TV camera is built. As illustrated in Fig. 1, when using the X-ray image receiving device 412a for the standing position, the X-ray tube 8 and the collimator 411 are oriented toward the X-ray image receiving device 412a for the standing position. When using the X-ray image receiving device 412b for the recumbent position, the X-ray tube 8 and the collimator 411 are oriented downward.

The operation table 407 includes an operation unit and a display unit. The operation unit receives the image-capturing condition or reproduction condition to be set thereto. The image-capturing condition is, for example, a condition at the time of the image-capturing such as a tube voltage and a tube current of the X-ray tube 8, image-capturing time and the like. The reproduction condition is an option such as selecting whether a series of captured images to be in a still image display or in a moving image display, selecting a reproduction speed (updating speed) of the moving images and the like. The operation unit is configured to include operation buttons, a mouse, a keyboard, a touch panel and the like. On the display unit, an operation screen is displayed to receive the optional image-capturing condition and the reproduction condition, and the operations by the mouse, the keyboard and the touch panel. In addition, reproduced still images and reproduced moving images are displayed on the display unit.

The control device 413 has a CPU and the storage device to be built therein and causes the CPU to read and execute a program which is stored in the storage device in advance to individually control each of the above-described devices, thereby executing the image-capturing while storing the images and the image-capturing conditions acquired during the image-capturing in the storage device. In addition, in response to the operation received by the operation unit of the operation table 407 from the operator, the control device 413 performs an image control such as reproducing desired cinema images at a desired reproduction speed. In addition, in the control device 413, a memory is built-in and the image-capturing condition and the captured images are stored.

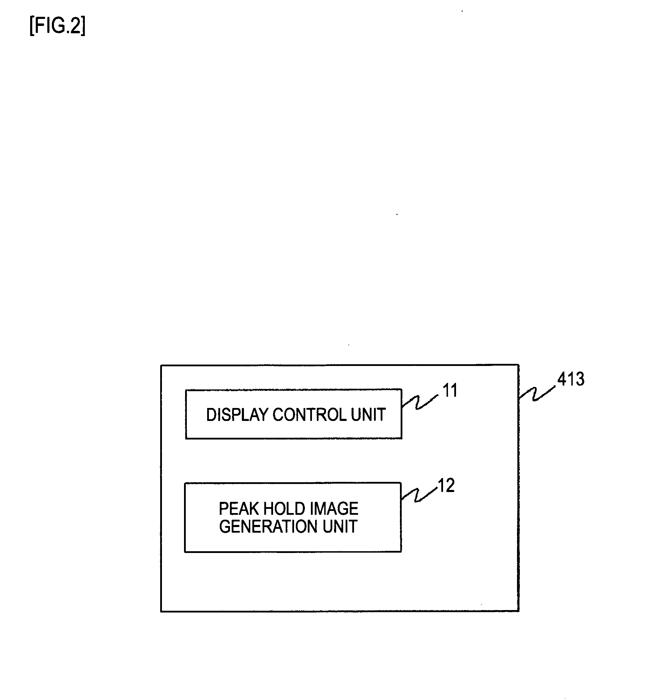

A function of an image control by the control device 413 is illustrated in Fig. 2. As in Fig. 2, the control device 413 has a display control unit 11 and a peak hold image generation unit 12. The display control unit 11 controls the images displayed on the display unit of the operation table 407.

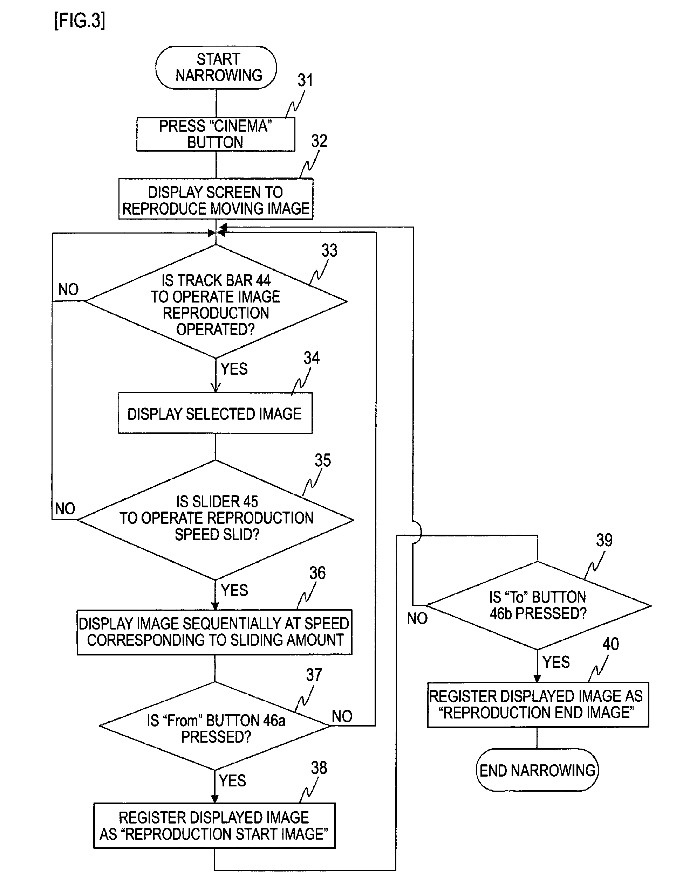

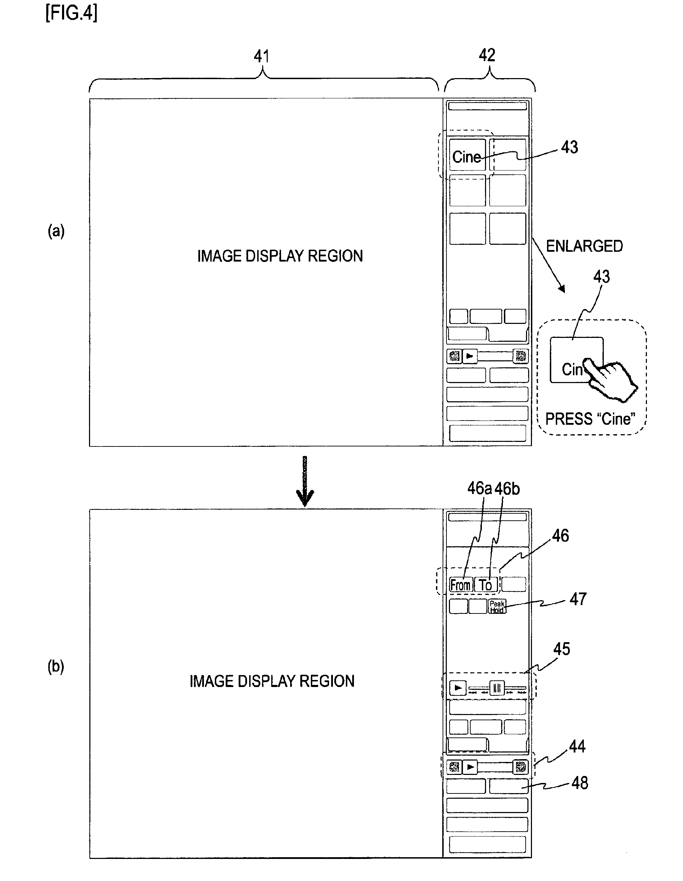

Next, an operation in the operation table 407 and the image control by the control device 413 will be described with reference to the drawing. Fig. 3 is a flow chart illustrating an operation of the operation table 407 and an operation of the image control. Fig. 4 is the display screen on the display unit of the operation table 407.

Here, the description will be made based on a state where a contrast agent is injected into a blood vessel of a test subject and consecutive images (also referred to as cinema images) consecutively obtained through the X-ray image receiving devices 412a and 412b as irradiating the X-ray from the X-ray tube 8 are stored in the storage device of the control device 413.

The display screen has an image display region 41 and an operation display region 42 in which various operation buttons and an operation slider are displayed. According to the embodiment, the display unit of the operation table 407 is configured to be in a touch panel structure, and the operator can select the operation by touching (pressing) the screen with the finger.

In the operation display region 42 in an initial screen, among the various operation buttons, a cinema (cine) button 43 is displayed by the display control unit 11 as illustrated in Fig. 4 (a). If the operator presses the cine button 43, the display screen (Fig. 4(b)) to reproduce the moving image is displayed by the display control unit 11 (steps 31 and 32). In the display screen (Fig. 4 (b)) to reproduce the moving image, in the operation display region 42, there are displayed multiple operation buttons including a track bar 44 to operate the image reproduction, a slider 45 to operate the reproduction speed, a reproduction start image and end image registration button 46, and a peak hold button 47.

According to the embodiment, it is possible to perform operational reproduction by designating the images within a desired range out of a series of images using the track bar 44 to operate the image reproduction, in other words, by narrowing the consecutive images within a desired range. Moreover, the operation of the track bar 44 to operate the image reproduction and the operation of the slider 45 to operate the reproduction speed are combined, and thus, it is possible to easily perform a complicated instruction such as a fine adjustment for the reproduction range and a fine adjustment for the reproduction speed.

Firstly, the narrowing operation of the reproduction image range in the consecutive images will be described.

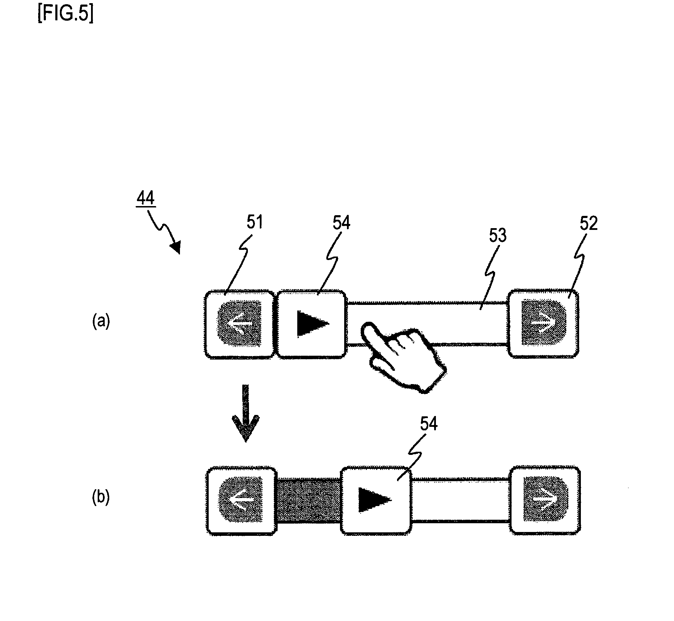

Figs. 5 (a) and 5 (b) are enlarged views of the track bar 44 to operate the image reproduction. The track bar 44 to operate the image reproduction includes a band-shaped region 53 with sending buttons 51 and 52 on which arrows are respectively marked pointing opposite each other to be disposed at both ends thereof, and a reproduction-stop button 54 capable of sliding on the band-shaped region 53. The reproduction-stop button 54 alternately switches between a reproduction state and a stop state every time it is pressed. In the stop state, when this button 54 is pressed, a triangle mark pointing to the right is displayed within the button as in Fig. 5(a) indicating that it is possible to receive the reproduction instruction. In the reproduction state, when this button 54 is pressed, a vertically double-lined mark is displayed within the button 54 indicating that it is possible to receive the stop instruction.

In the band-shaped region 53, a length direction thereof corresponds to an overall time axis of a series of the consecutive images (cinema images) which are stored in the storage device of the control device 413. In other words, the left end of the band-shaped region 53 corresponds to the beginning image in the consecutive images, and the right end corresponds to the last image in the consecutive images.

The operator presses a desired position of the band-shaped region 53 with the finger, thereby selecting an image corresponding to the pressed position (step 33). The display control unit 11 reads out image data corresponding to the pressed position of the band-shaped region 53 from consecutive image data in the storage device, thereby the display control unit 11 displays the image on the image display region 41. At the same time, the reproduction-stop button 54 is moved to the pressed position, and a color of the band-shaped region 53 on the left side of the reproduction-stop button 54 is changed to a predetermined color, thereby notifying the operator of the pressed position (step 34).

Accordingly, since the operator can skip the image in the beginning portion in the consecutive images in which no contrast agent flows in the blood vessel yet in the image-capturing region to display the image designated by the operator, it is possible to search for and display the first image showing the contrast agent.

The sending button 51 or 52 is pressed, and thus, it is possible to shift the selected image one by one to an image in each direction of the beginning and the end by the number of button presses. Therefore, it is possible that the reproduction-stop button 54 moves to the left side/right side in response to the number of button presses as well by pressing the sending button 51 or 52. Accordingly, it is possible to search for the first image in which the contrast agent is shown.

However, it is not easy to find the first image in which the contrast agent is shown with only this adjustment. Therefore, after aiming at an approximate target through the above-described operation, the first image in which the contrast agent is shown is further searched for using the slider 45 to operate the reproduction speed.

The slider 45 to operate the reproduction speed includes a reproduction-stop button 61, a slider button 63, a band-shaped region 62, and an arrow display 64 indicating a speed of shifting the selected image.

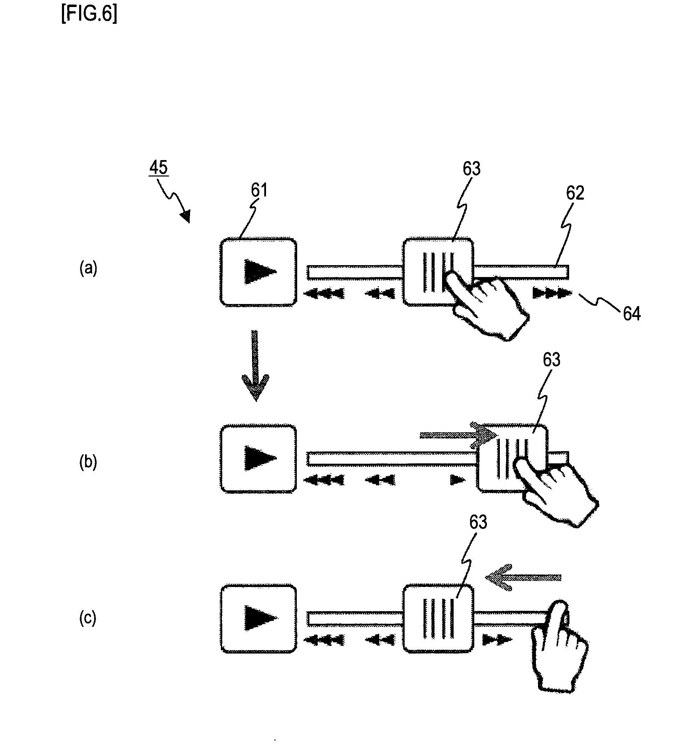

The reproduction-stop button 61 is alternately switched between the reproduction state and the stop state every time it is pressed. In the stop state, when this button 61 is pressed, the triangle mark pointing to the right is displayed within the button 61 as in Fig. 6(a) indicating that it is possible to receive the reproduction instruction. In the reproduction state, when this button 61 is pressed, the vertically double-lined mark is displayed within the button indicating that it is possible to receive the stop instruction. During the narrowing operation, the reproduction-stop button 61 is not pressed.

If the operator causes the slider button 63 to slide to the right or left along the band-shaped region 62 as in Figs. 6(a) and 6(b), the display control unit 11 consecutively displays the current images on the image display region 41 in an ending direction or a beginning direction of the consecutive images one by one as speeding up to a speed corresponding to a sliding amount (step 35). In other words, if the sliding amount is large, the consecutive display is performed in a very fast sending, and if the sliding amount is small, the consecutive display is performed slowly.

When the operator moves the hand off the slider button 63, the display control unit 11 causes the slider button 63 to return to the center position as illustrated in Fig. 6(c), thereby stopping the movement of the image (step 36).

Accordingly, it is possible to consecutively display the images which are in the periphery of the image selected through the track bar 44 on the image display region at a speed corresponding to the sliding amount of the slider button 63. Therefore, the operator can check the consecutive images displayed on the image display region 41 and find the image in which the contrast agent is shown.

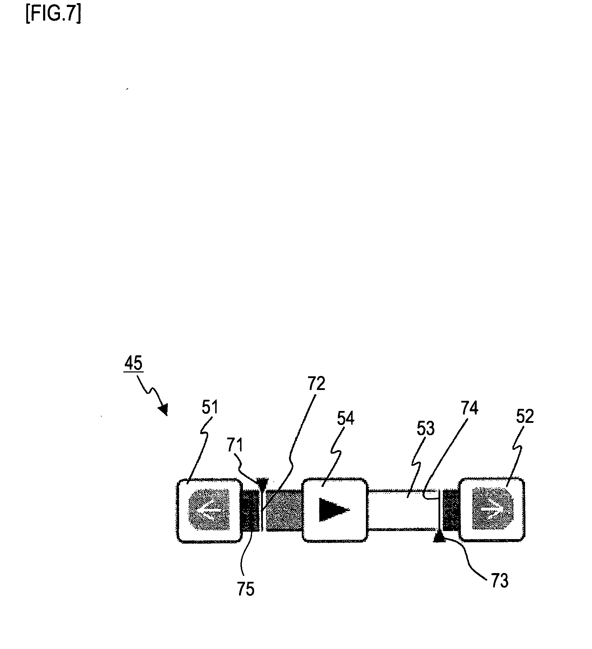

The reproduction start image and end image registration button 46 includes a "From" button 46a and a "To" button 46b. The operator presses the "From" button 46a when sighting the first image in which the contrast agent is shown through the above-described operation (step 37). Accordingly, the display control unit 11 registers this image data as a "reproduction start image". Specifically, the image data is stored in the storage device by setting a flag and the like. In addition, as in Fig. 7, the display control unit 11 displays an arrow mark 71 and a vertical line 72 which indicate a position corresponding to the reproduction start image of the band-shaped region 53 of the track bar 44 to operate the image reproduction (step 38).

1 Similarly, when the last image in which the contrast agent is shown is sighted by repeating steps 33 to 36, the operator presses the "To" button 46b (step 39). Accordingly, display control unit 11 stores the image data as the "reproduction end image" in the storage device by setting the flag and the like. In addition, the display control unit 11 displays an arrow mark 73 and a vertical line 74 which indicate a position corresponding to the reproduction end image of the band-shaped region 53 of the track bar 44 to operate the image reproduction (step 38). Moreover, with respect to a region between the vertical line 71 and the vertical line 74, an outer side thereof is displayed being lowered in brightness, thereby displaying the narrowed reproduction range and the non-reproduction range to be easily recognized.

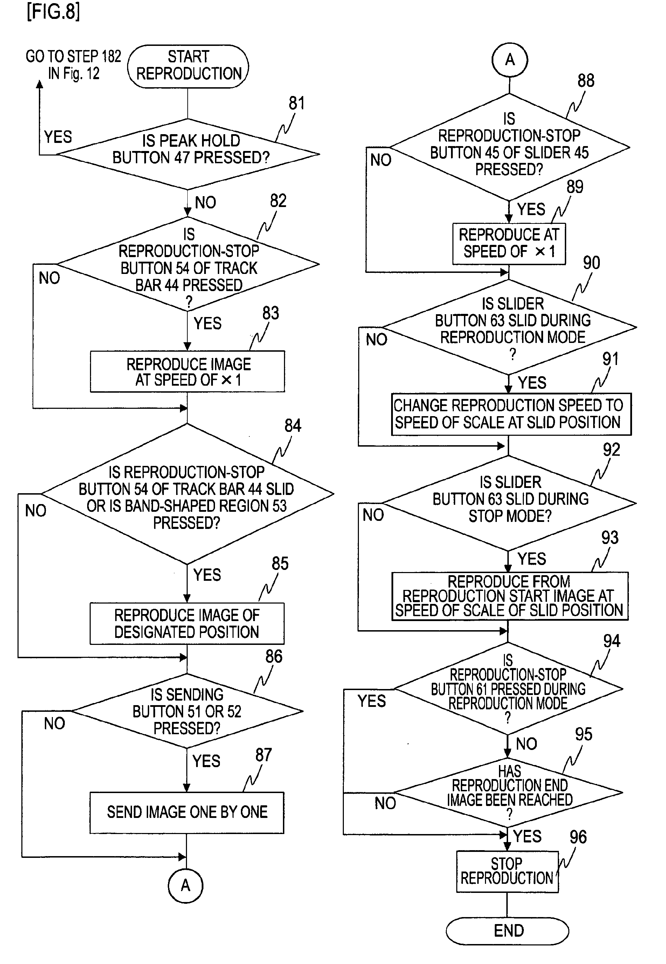

According to the above-describe operation, if the range to be reproduced is narrowed out of a series of cinema images, it is possible to generate the peak hold image to be displayed while performing the moving display and reproduction with the images in the narrowed range. This operation will be described below with reference to a flow in Fig. 8.

The operator can perform the reproduction or the peak hold image display while reproducing by any operation of six types below. Firstly, a reproduction operation in which peak hold processing is not performed will be described.

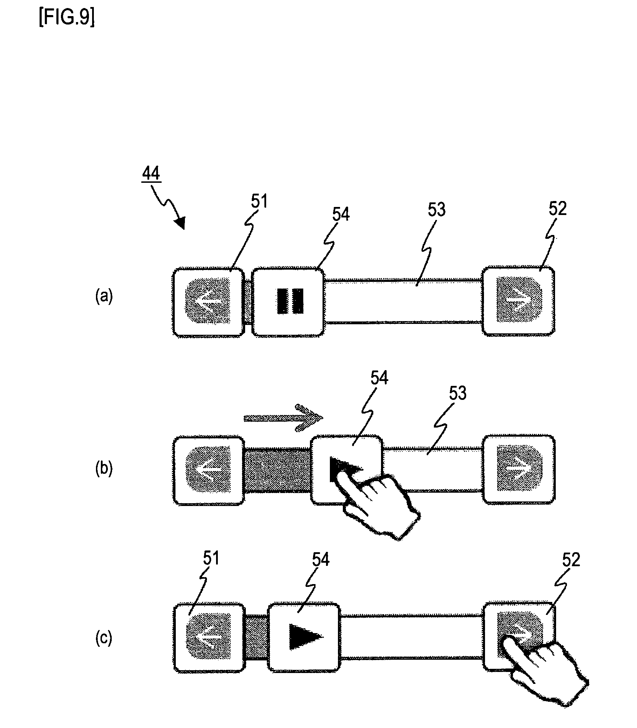

In a case where the reproduction-stop button 54 of the track bar 44 to operate the image reproduction is pressed in a state where peak hold button 47 is not pressed by the operator (step 81), the display control unit 11 sequentially reads out the images from the beginning "reproduction start image" within the reproduction range which are already narrowed and displays the same on the image display region 41 at a speed of ×1 (steps 82 and 83). Accordingly, as a mark within the reproduction-stop button 54, the vertically double-lined mark is displayed as in Fig. 9(a) indicating that it is possible to receive the stop instruction.

In addition, when the operator presses a desired position in the band-shaped region 53 of the track bar 44 to operate the image reproduction as in Fig. 9(b), the image of the position designated by the pressing is promptly displayed, and the images in the middle are skipped so as not to be displayed. In addition, when the reproduction-stop button 54 is slid to a desired position, the images in the slid range are sequentially displayed in response to the slide speed (steps 84 and 85).

In addition, as in Fig. 9(c), when the operator presses the right-directed sending button 52, the images are displayed one by one being sequentially sent on each pressing (steps 86 and 87). In addition, when the left-directed sending button 51 is pressed, the images are displayed one by one being reversely sent on each pressing.

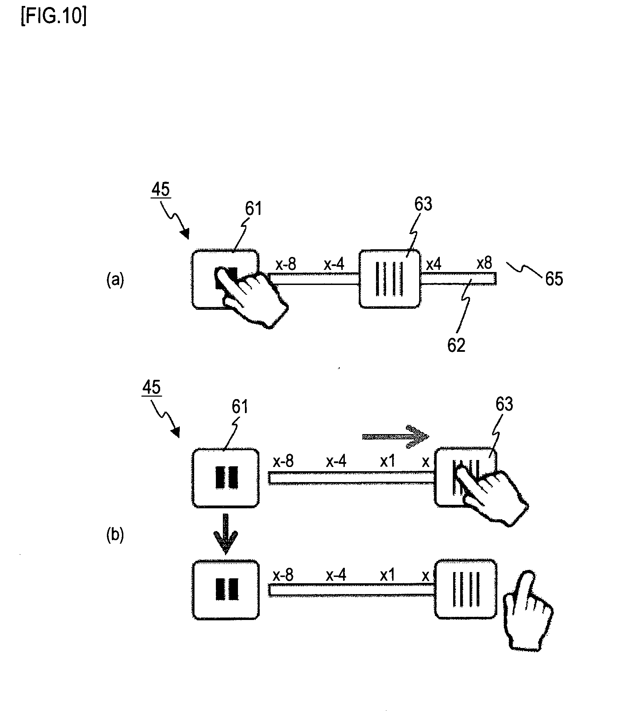

In addition, as in Figs. 10(a) and 10(b), the operator can perform the reproduction by operating the slider 45 to operate the reproduction speed. In other words, as in Fig. 10(a), the operator presses the reproduction-stop button 61 of the slider 45 to operate the reproduction speed, the display control unit 11 reproduces the images at the speed of ×1 (steps 88 and 89) . As a mark within the reproduction-stop button 61, the vertically double-lined mark is displayed as in Fig. 10 (a) indicating that it is possible to receive the stop instruction. During a reproduction mode, a scale display 65 ("×1", "x4", "×8", "×-4", "x-8") is displayed on an upper portion of the band-shaped region 62 indicating the reproduction speed and the reproduction direction. "×1", "x4" and "x8" respectively represent ×1 reproduction, x4 reproduction and ×8 reproduction. "x-4" and "x-8" respectively represent reverse x4 reproduction and reverse x8 reproduction. If the reproduction-stop button 61 is pressed to start the ×1 reproduction, the display control unit 11 moves the slider button 63 to the "×1" position in the scale display 65, thereby notifying the operator of ×1 reproduction.

As in Fig. 10 (b), if the operator slides the slider button 63 during the reproduction mode, the display control unit 11 reproduces the images causing the reproduction speed to be a speed corresponding to the scale display 65 of the slid position (steps 90 and 91). The slider button 63 stays at the slid position, thereby continuing the reproduction at that speed.

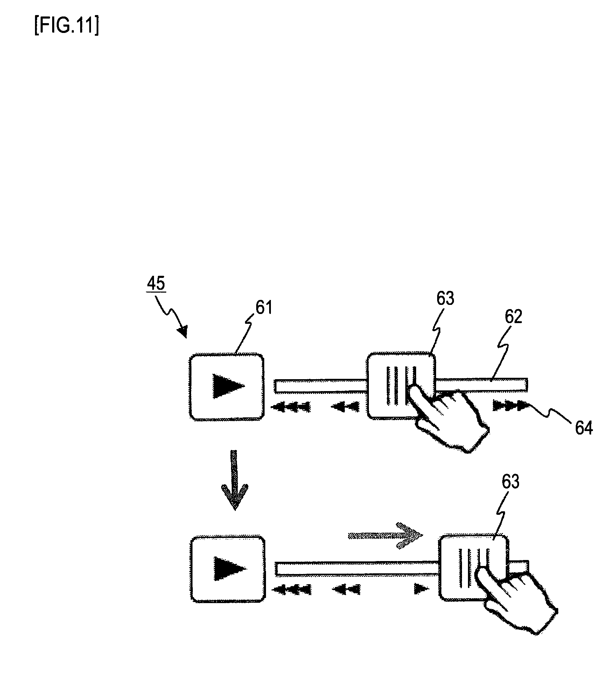

In addition, as in Fig. 11, when the operator slides the slider button 63 as maintaining the stop state without pressing the reproduction-stop button 61 of the slider 45 to operate the reproduction speed, the display control unit 11 starts the reproduction from "reproduction start image" in the narrowed image range at the reproduction speed of the scale of the slid position (steps 92 and 93) . When the operator moves the hand off the slider button 63, the display control unit 11 causes the slider button 63 to return to the original position, thereby stopping the reproduction.

While the reproduction is performed through the above-described steps 82 to 91, if the reproduction-stop button 54 or 61 is pressed, the reproduction stops (steps 94 and 96) . In addition, in a case where the images reach the "reproduction end image" which is registered by narrowing during the reproduction mode through each of the above-described steps, the reproduction also ends. Accordingly, the images after the "reproduction end image" are not reproduced, and thus, it is possible to reproduce only the image in the range narrowed by the operator. In above description, an example in which the reproduction ends even in a case of reaching the "reproduction end image" is described. However, without being limited thereto, it is possible to be configured in loop reproduction in a case of reaching the "reproduction end image".

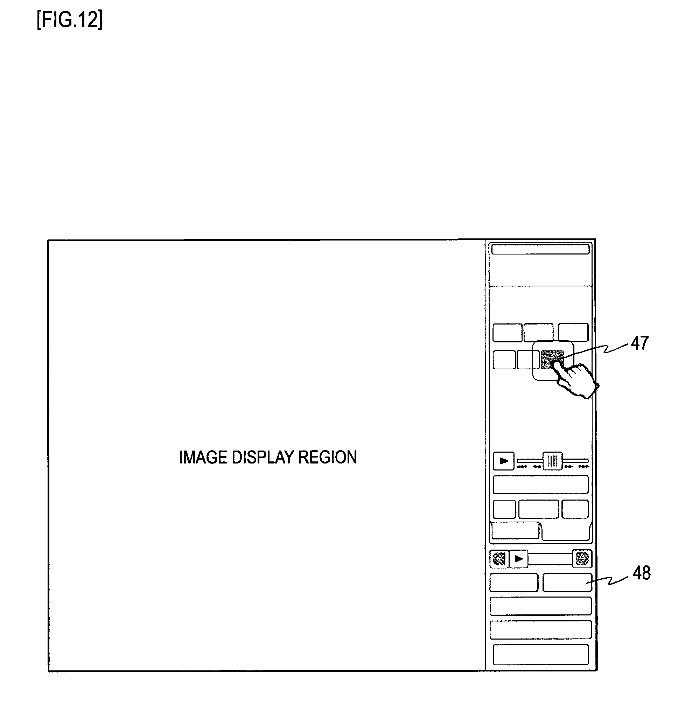

The above-described steps 82 to 95 illustrate general reproduction without performing the peak hold. However, in step 81 of Fig. 8, in a case where the peak hold button 47 is pressed as in Fig. 12, successive peak hold image generation is processed while reproducing, thereby successively displaying the peak hold images by that time on the image display region 41. The generation process of the peak hold image is generated by the peak hold image generation unit 12 inside the control device 413.

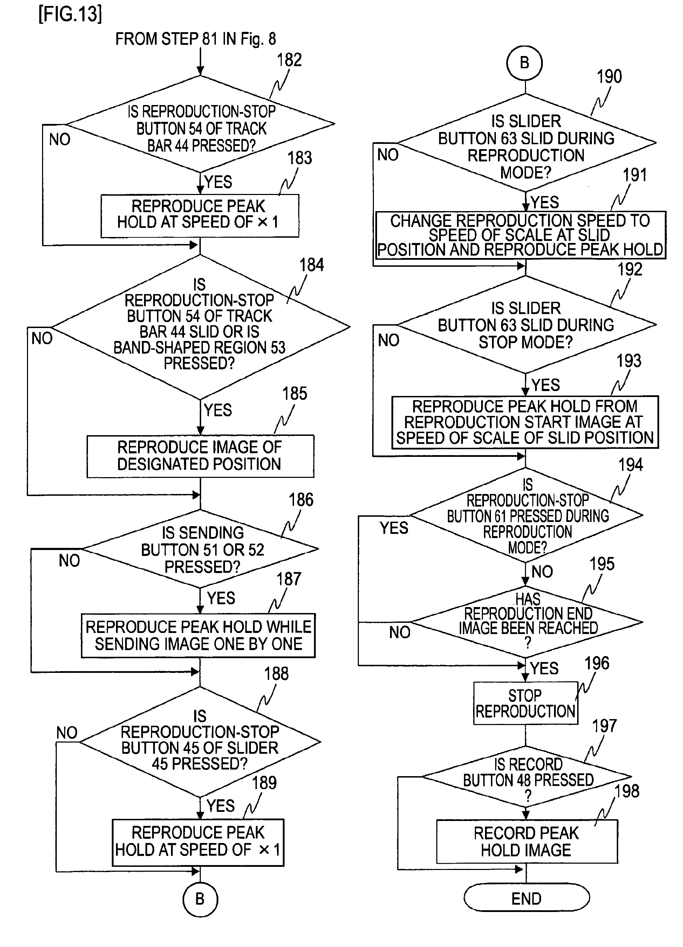

In other words, if the peak hold button 47 is pressed in step 81 prior to step 82 in Fig. 8, the step is advanced to steps 182 to 196 in Fig. 13. Steps 182 to 197 respectively correspond to steps 82 to 97 in Fig. 8. Similar to steps 82 to 97, the reproduction images are selected using the track bar 44 to operate the image reproduction and the slider 45 to operate the reproduction speed, thereby setting the reproduction speed. However, since the peak hold button 47 is pressed, in steps 183, 185, 186, 187, 189, 191 and 193, the display control unit 11 not only simply displays the image but also sequentially transfers the image data read out to be reproduced to the peak hold image generation unit 12 and sequentially performs adding, thereby generating the peak hold image. A method of processing the peak hold image generation is widely known so that the detailed description will be omitted herein. The display control unit 11 sequentially displays the generated peak hold images. In step 184, in a case where the operator presses a desired position in the band-shaped region 53 of the track bar 44 to operate the image reproduction, since the image of the position designated by the pressing is promptly displayed and the images in the middle are skipped so as not to be displayed, the skipped images not to be displayed are not transferred to the peak hold image generation unit 12, thereby not performing the adding. In addition, when the reproduction-stop button 54 is slid to a desired position, the images in the range of sliding are sequentially displayed, thereby performing the adding by the peak hold image generation unit 12.

The displayed peak hold images are stored in the storage device inside the control device 413 by pressing a preservation button 48 of the operation display region 42 by the operator (steps 197 and 198).

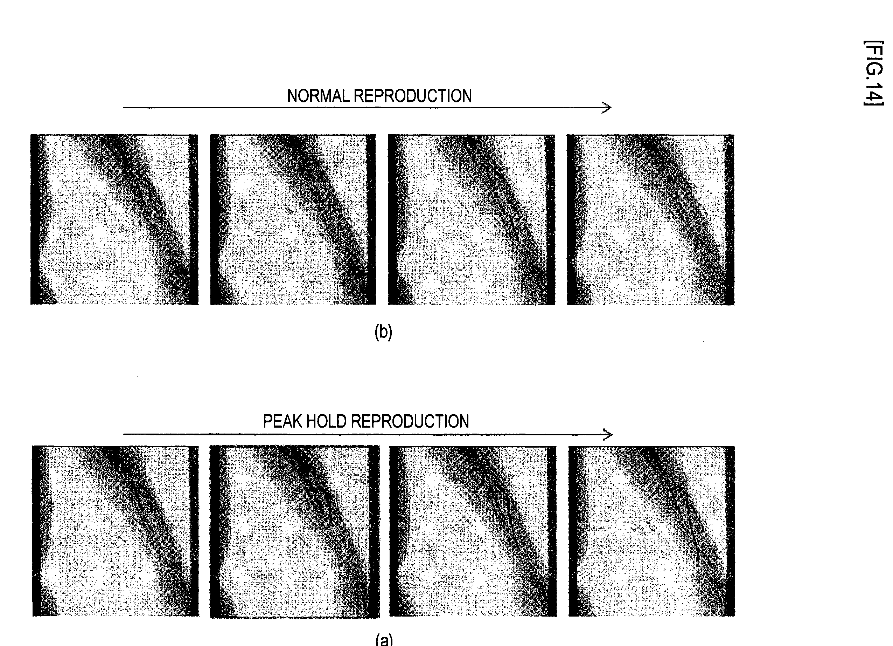

Fig. 14(a) illustrates images reproduced through steps 82 to 96 in Fig. 8 at a predetermined time intervals. With reference to Fig. 14 (a), it is possible to observe a state where the contrast agent flows in the blood vessel to move to a peripheral portion. Fig. 14(b) illustrates the same images as Fig. 14(a) which are reproduced while performing the peak hold through the steps 182 to 196 in Fig. 13 at a predetermined time intervals. In Fig. 14(b), since the contrast agent is added by the peak hold processing, it is possible to understand the overall shape of the blood vessel image as time passes.

In this manner, according to the embodiment, the track bar 44 to operate the image reproduction and the slider 45 to operate the reproduction speed are combined, and thus, it is possible for the operator to narrow a series of the consecutive images into an intended reproduction range to perform the reproduction at a desired reproduction speed or the peak hold reproduction. Accordingly, it is possible to omit unnecessary images in which the contrast agent is not shown and perform the reproduction in a short period or the peak hold reproduction. For this reason, even though the operator repeatedly performs the reproduction several times as changing the reproduction speed or the state whether or not the peak hold is present, it is possible to make the overall reproduction time short.

In addition, it is possible to provide multiple operation methods as a setting operation for the narrowing, reproducing and reproduction speed by combining the track bar 44 to operate the image reproduction and the slider 45 to operate the reproduction speed. Accordingly, the operator can select an easy method to be operated and operate, and thus, it is possible to easily perform the narrowing operation and the reproduction operation in a short time.

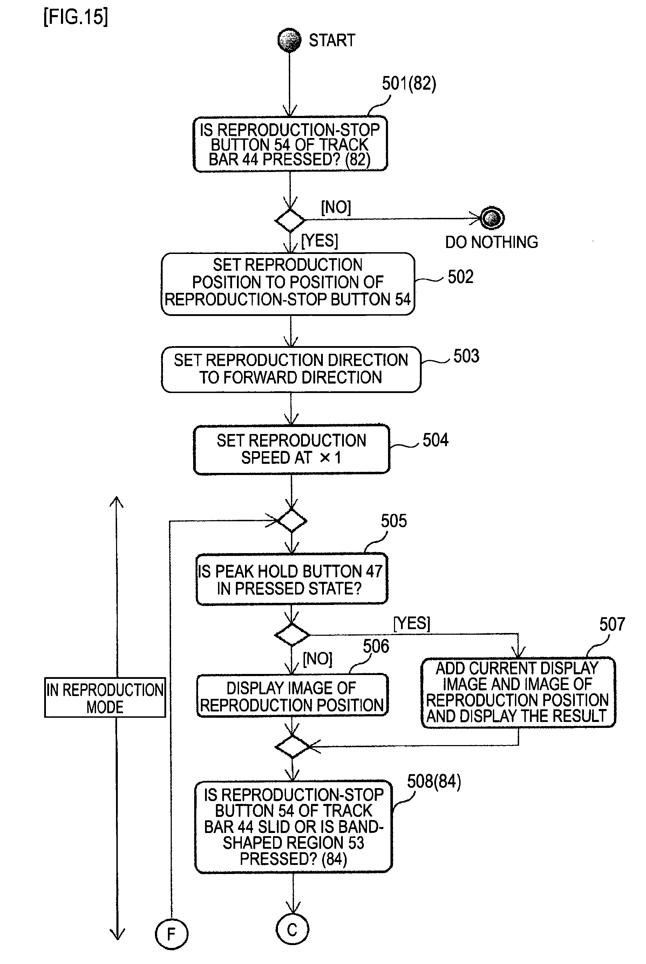

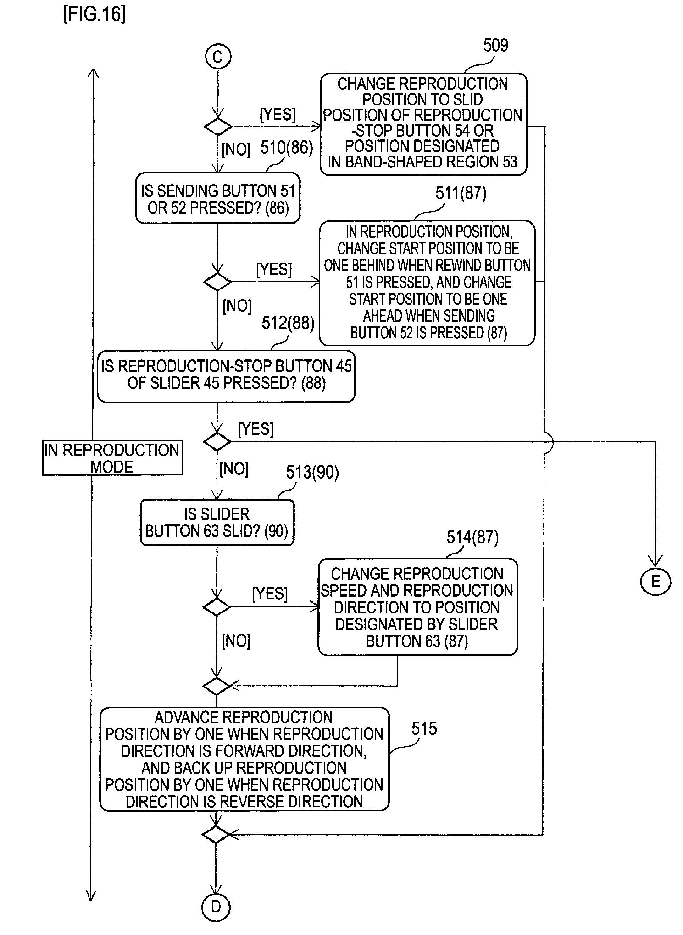

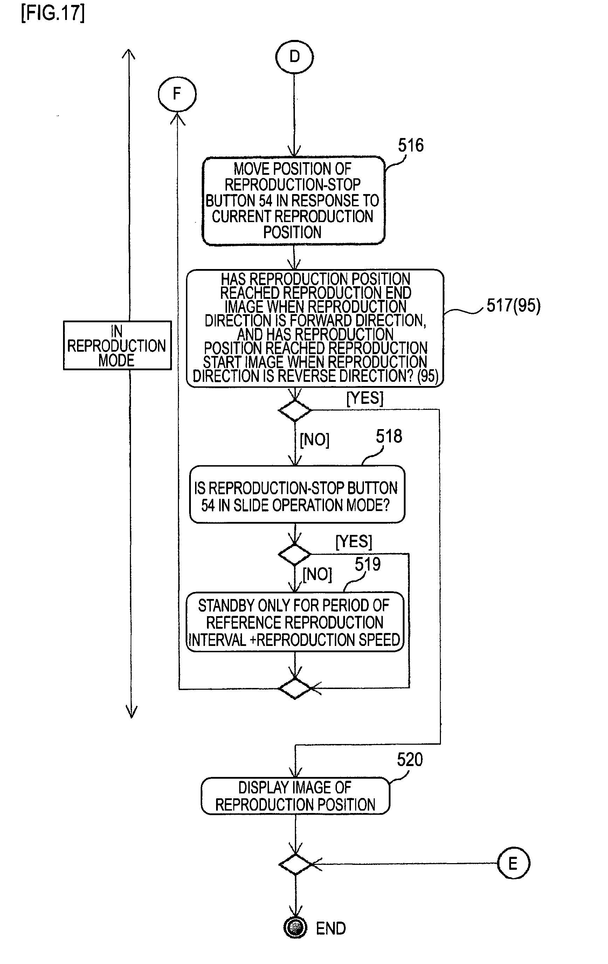

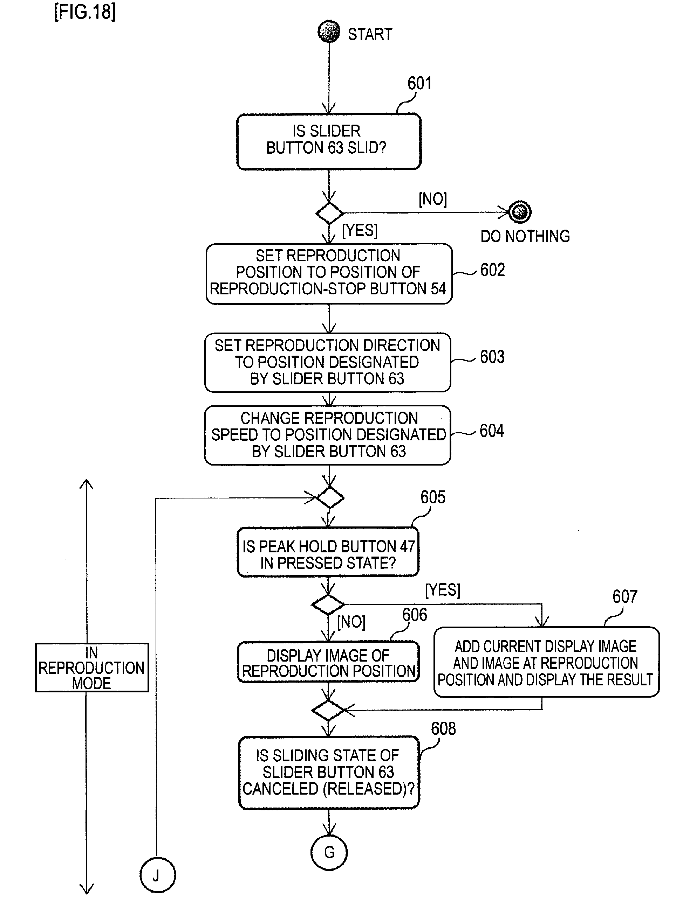

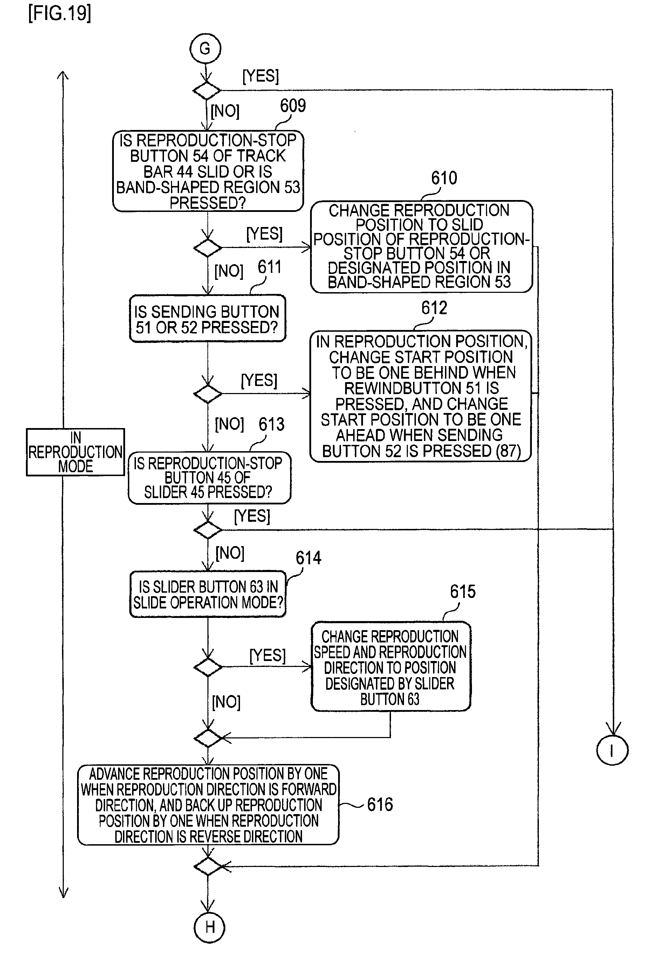

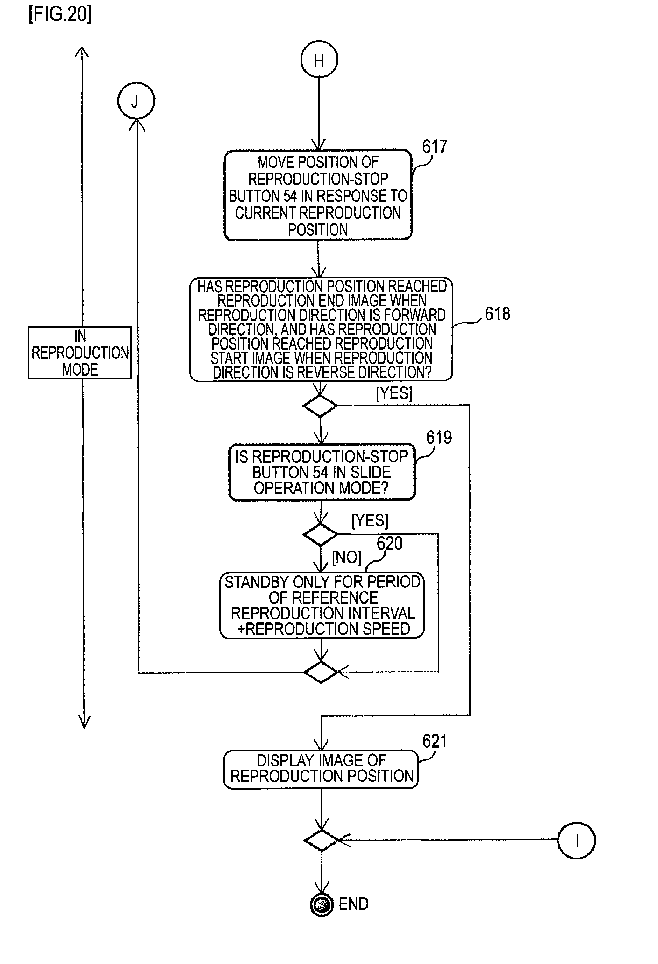

The processing operations of the reproduction display and the peak hold image display while reproducing by using the display control unit 11 can be performed in a different processing operation without being limited to the flows in Figs. 8 and 13. For example, it is possible to adopt the flow of steps 501 to 520 in Figs. 15 to 17 or the flow of steps 601 to 621 in Figs. 18 to 20. The flow throughout Figs. 15 to 17 is a flow in a case of starting the reproduction by pressing the reproduction-stop button 54 of the track bar 44. The flow throughout Figs. 18 to 20 is a flow in a case of starting the reproduction by sliding the slider button 63. In the flow of Figs. 15 to 17, the step numbers shown in parenthesis indicate the corresponding step in Fig. 13. Both of the flow of steps 501 to 520 in Figs. 15 to 17 and the flow of steps 601 to 621 in Figs. 18 to 20 are enabled to handle a real-time operation during the reproduction mode.

### Explanation of Reference

8: X-ray tube, 11: display control unit, 12: peak hold image generation unit, 41: image display region, 42: operational display region, 43: cinema button, 44: track bar to operate image reproduction, 45: slider to operate reproduction speed, 46: reproduction start image and end image registration button, 51 and 52: sending buttons, 53: band-shaped region, 54: reproduction-stop button, 61: reproduction-stop button, 62: band-shaped region, 63: slider button, 64: arrow display

## Claims

1. Diagnostic X-ray imaging equipment comprising:
a storage device that stores X-ray images continuous in chronological order; and
a display control unit that controls reproduction of the X-ray images,
wherein the display control unit causes a first band-shaped region of which an overall length corresponds to an overall length of time of consecutive X-ray images and a second band-shaped region in which a plurality of positions in a length direction respectively correspond to a plurality of types of reproduction speed determined in advance to be displayed on a display device,
in a case where an operator designates a position in the first band-shaped region, the display control unit selects an X-ray image corresponding to the designated position among the consecutive X-ray images to display on the display device, and
in a case where the operator designates a position in the second band-shaped region, the display control unit reads out consecutive X-ray images in chronological order among the X-ray images which are selected to be displayed, at a speed corresponding to the designated position, to display on the display device.

2. The diagnostic X-ray imaging equipment according to Claim 1,
wherein the display control unit causes a reproduction start image registration button and a reproduction end image registration button which register consecutive images within a predetermined time range among the X-ray images continuous in chronological order to be displayed on the display device,
in a case where the operator operates the reproduction start image registration button, the display control unit causes an X-ray image which is displayed on the display device at that time to be registered with the storage device as a reproduction start image of the consecutive images within the predetermined time range, and
in a case where the operator operates the reproduction end image registration button, the display control unit causes an X-ray image which is displayed on the display device at that time to be registered with the storage device as a reproduction end image of the consecutive images within the predetermined time range.

3. The diagnostic X-ray imaging equipment according to Claim 2,
wherein the display control unit causes a reproduction button which instructs reproduction to be displayed on the display device,
in a case where the operator operates the reproduction button, the display control unit sequentially reproduces reproduction images from the reproduction start image through the reproduction end image which are registered in the storage device, and
during this reproducing, when the operator designates a position in the second band-shaped region, the display control unit changes a reproduction speed to a speed corresponding to the designated position.

4. The diagnostic X-ray imaging equipment according to Claim 1,
wherein the display control unit causes a slider button for the operator designating a position in the second band-shaped region to be displayed, and
in a case where the operator continues an operation designating the position in the second band-shaped region using the slider button, the display control unit causes the slider button to stop at the designated position, and in a case where the operator finishes the operation of the slider button, the display control unit causes the slider button to return to a position determined in advance in the second band-shaped region.

5. The diagnostic X-ray imaging equipment according to Claim 3,
wherein the display control unit displays predetermined marks at positions in the first band-shaped region respectively corresponding to a time position of the reproduction start image and the reproduction end image.

6. The diagnostic X-ray imaging equipment according to Claim 3,
wherein the display control unit causes a second reproduction button with which the operator instructs reproduction to be displayed in the first band-shaped region, and
the display control unit moves the second reproduction button to a position in the first band-shaped region corresponding to a time position of the X-ray image which is currently displayed on the display device.

7. The diagnostic X-ray imaging equipment according to Claim 2, further comprising:
a peak hold image generation unit that generates a peak hold image by sequentially adding the X-ray images,
wherein the display control unit causes a peak hold button which receives an instruction to generate the peak hold image from the operator to be displayed on the display device,
in a case where the operator operates the peak hold button, the display control unit causes reproduction images from the reproduction start image through the reproduction end image to be sequentially transferred to the peak hold image generation unit and causes the peak hold images by that time to be sequentially generated, and
the display control unit causes the generated peak hold images to be sequentially displayed.

8. A method of displaying X-ray images continuous in chronological order,
wherein a first band-shaped region of which an overall length corresponds to an overall length of time of the consecutive X-ray images and a second band-shaped region in which a plurality of positions in a length direction respectively correspond to a plurality of types of reproduction speed determined in advance are displayed on a display device,
in a case where an operator designates a position in the first band-shaped region, an X-ray image corresponding to the designated position is selected among the consecutive X-ray images to be displayed on the display device, and
in a case where the operator designates a position in the second band-shaped region, consecutive X-ray images are read out in chronological order among the X-ray images which are selected to be displayed at a speed corresponding to the designated position to be displayed on the display device.
